# EUROPÄISCHE PATENTSCHRIFT

(11) **EP 0 711 812 B1**
(45) Veröffentlichungstag und Bekanntmachung des Hinweises auf die Patenterteilung: **29.08.2001**
(21) Anmeldenummer: 95810688.2
(22) Anmeldetag: 01.11.1995
(51) Int. Cl.: C09B 5/62, C08K 5/34, C09D 11/02, C09K 11/06, G03G 5/06

(54) **Verfahren zur Herstellung von Perylenimiden, neue di-, tri- und tetrachromophore Perylenfarbstoffe und deren Verwendung**
Method of preparation of perylene imides, novel di-, tri-, and tetrachromophore perylene dyestuffs and their utilization
Procédé de préparation de pérylène-imides, nouveaux colorants de di-, tri-, et tetrachromophore pérylènes et leur utilisation

(30) Priorität: 10.11.1994 CH 337694
(43) Veröffentlichungstag der Anmeldung: 15.05.1996
(73) Patentinhaber: Ciba Specialty Chemicals Holding Inc., 4057 Basel (CH)
(72) Erfinder: Langhals, Heinz, Prof. Dr., D-85521 Ottobrunn (DE)

(56) Entgegenhaltungen:
- EP-A- 0 443 566
- DE-A- 4 018 830
- GB-A- 1 370 433
- US-A- 4 968 571

## Beschreibung

Die Erfindung betrifft neue di-, tri- und tetrachromophore Perylenfarbstoffe, ein Verfahren zu deren Herstellung durch Umsetzung von Perylen-3,4:9,10-tetracarbonsäure-3,4-anhydrid-9,10-imiden mit Formamiden der entsprechenden primären Di-, Tri- oder Tetraamine, und deren Verwendung, sowie ein spezifisches Verfahren zur Herstellung von Perylen-3,4:9,10-tetracarbonsäure-bisimiden durch Umsetzung von Perylen-3,4:9,10-tetracarbonsäure-bisanhydriden oder von Perylen-3,4:9,10-tetracarbonsäure-anhydrid-imiden mit Formamiden primärer Amine.

Perylenfarbstoffe, Perylen-3,4:9,10-tetracarbonsäurebisimide, werden üblicherweise aus dem technisch gut zugänglichen Perylen- 3,4:9,10-tetracarbonsäurebisanhydrid oder Perylen-3,4:9,10-tetracarbonsäure-3,4-anhydrid-9,10-imiden und aus primären aliphatischen oder aromatischen Aminen dargestellt.

Während die Umsetzung bei gewöhnlichen primären Aminen unter den üblichen Reaktionsbedingungen glatt gelingt, erfolgt die Umsetzung mit besonders elektronenreichen Aminen, insbesondere mit elektronenreichen aromatischen Aminen, wie z.B. dem 1,3,5-Triaminobenzol, zu anderen als den erwarteten Produkten. Die glatte Umsetzung dieser Amine mit Perylen-peri-Anhydrid-Einheiten ist ein ungelöstes synthetisches Problem. Die elektronenreichen aromatischen oder heteroaromatischen Amine sind darüber hinaus im allgemeinen sehr oxydationsempfindlich, insbesondere in Gegenwart von Basen, was ihre Handhabung erheblich erschwert. Beispiele sind Amine, die eine oder mehrere Amino-Gruppen, Alkoxy-Gruppen, Phenoxy-Gruppen tragen und/oder sich von elektronenreichen Heteroaromaten wie Pyrrol oder Furan ableiten. Ein Weg, die Oxydationsempfindlichkeit dieser Amine, insbesondere in Gegenwart von Basen, zu verringern, z.B. durch eine Derivatisierung, wäre daher für die Synthese der Perylenfarbstoffe ein erheblicher Fortschritt.

Werden für die Kondensation von Perylen-3,4:9,10-tetracarbonsäure-bisanhydrid oder- monoanhydrid-monoimiden statt der primären Amine nun die Formamide der betreffenden Amine eingesetzt, so findet man unter den üblichen Reaktionsbedinungen erstaunlicherweise eine glatte Umsetzung zu den Bisimiden. Die Formamide reagieren dabei unter Entwicklung von CO₂ (nach Oxydation) zu den gleichen Produkten als würde man die entsprechenden freien Amine für die Reaktion einsetzen.

Obwohl die Umsetzung von Carbonsäureanhydriden, wie z.B. Phthalanhydrid, Naphthalindicarbonsäureanhydrid, Trimellithsäureanhydrid oder Pyrromelithsäuredianhydrid, mit Formamid oder mit N-Methylformamid im allgemeinen bekannt ist [siehe z.B. H. Schindlbauer, Monatshefte für Chemie 104, 848(1973)], ist diese Reaktion bisher nur einmal, in der GB-B 1,370,433, für die Herstellung eines bestimmten Perylentetracarbonsäure-bisimids beschrieben worden.

GB-A 1,370,433 beansprucht ein Verfahren zur Herstellung von N,N'-Dimethylperylen-3,4,9,10-tetracarbonsäurediimid durch Reaktion von Perylen-3,4,9,10-tetracarbonsäuredianhydrid mit Methylamin oder einer Verbindung, die Methylamin *in situ* liefert wie N-Methylformamid. Das Verfahren wird in wässriger Lösung ohne ein zusätzliches organisches Lösungsmittel oder in der Methylamin-liefernden Verbindung durchgeführt.

Bei der erfindungsgemässen Umsetzung der Formamide werden diese nicht, wie man zunächst vermuten könnte, und wie in der oben erwähnten GB-B 1,370,433 für die Synthese von N,N'-Dimethylperylen-3,4:9,10-tetracarbonsäure-bisimid in wässrigen Lösungen beschrieben ist, zu den freien Aminen verseift, die dann, wie üblich, abreagieren könnten. Vielmehr liegt hier ein neuer Reaktionstyp vor - die Formamid-Gruppierung greift aktiv in das Reaktionsgeschehen ein. Ein Beleg hierfür ist die erhöhte Reaktivität der Formamide verglichen mit den Aminen, wie auch die im allgemeinen verbesserte Ausbeute bei der Reaktion mit den Formamiden. So können aromatische Formamide glatt umgesetzt werden, auch wenn die entsprechenden Amine nicht oder nur sehr langsam reagieren.

Die Verwendung von Formamiden statt der betreffenden Amine hat in bestimten Fällen erhebliche Vorteile. So sind die Formamide im allgemeinen gegenüber den freien Aminen völlig lagerbeständig, während bei den letzteren die Oxydation in vielen Fällen ein erhebliches Problem darstellt (vgl. z.B. die bekannte Braunfärbung bei der Lagerung von Anilin, wenn nicht auf völlige Abwesenheit von Sauerstoff geachtet wird). Ein ganz wesentlicher Fortschritt wird aber erreicht, wenn das primäre Amin sehr elektronenreich ist. So bereitet die Umsetzung von 1,3,5-Triaminobenzol mit dem N-(1-Hexylheptyl)perylen-3,4:9,10-tetracarbonsäure-3,4-anhydrid-9,10-imid wegen der Oxydationsempfindlichkeit des Amins erhebliche Probleme. Erfolgt dagegen die Kondensation mit dem Tris-Formamid des 1,3,5-Triaminobenzols, so reagieren alle drei Aminogruppen glatt, und es wird der entsprechende trichromophore Farbstoff erhalten, der der erste Perylenfarbstoff mit Y-förmiger Anordnung der Chromophore ist.

Der trichromophore Perylenfarbstoff hat das übliche Absorptions- und Fluoreszenzspektrum, er weist aber eine schnelle intramolekulare Energieübertragung auf - dies hat Folgen in bezug auf die Fluoreszenzdepolarisation, so dass Farbstoffe dieses Typs analytisch problemlos neben monofluorophoren Perylenfarbstoffen über den Lineardichroismus nachgewiesen bzw. analytisch bestimmt werden können.

Setzt man dagegen für die Umsetzung mit N-(1-Hexylheptyl)perylen-3,4:9,10-tetracarbonsäure-3,4-anhydrid-9,10-imid beispielsweise das freie nicht formamidderivatisierte Triaminopyrimidin ein, so erhält man nicht den trichromophoren sondern nur den dichromophoren Perylenfarbstoff.

Di- beziehungsweise tri- und tetrachromophore Perylenfarbstoffe sind bisher kaum, beziehungsweise noch gar nicht, beschrieben worden. Zum Stand der Technik gehören einzig zwei Vertreter der dichromophoren Farbstoffe, welche beide auf konventionelle Art, durch Umsetzung eines Perylen-3,4:9,10-tetracarbonsäure-3,4-anhydrid-9,10-imids mit dem freien primären Diamin hergestellt wurden.

H. Kaiser, J. Lindner und H. Langhals beschreiben in Chem. Ber. 124, 529 (1991) die Synthese von nichtsymmetrisch substituierten Perylen-Fluoreszenzfarbstoffen. Dabei wird auch die Herstellung des dichromophoren Produktes der Umsetzung von 1,4-Diaminobutan mit N-(2,5-Di-tert-butylphenyl)-perylen-3,4:9,10-tetracarbonsäure-monoanhydrid-monoimid beschrieben.

Die US 4,968,571 und die EP-A 443,566 beschreiben die Verwendung von photoleitfähigen Perylenderivaten in elektrophotographischen Aufzeichnungsmaterialien. Dabei wird auch das dichromophore Produkt der Umsetzung von 1,4-Phenylendiamin mit N-Phenethyl--perylen-3,4:9,10-tetracarbonsäure-monoanhydrid-monoimid erwähnt.

Gegenstand vorliegender Anmeldung sind Perylen-3,4:9,10-tetracarbonsäureimide der Formel I 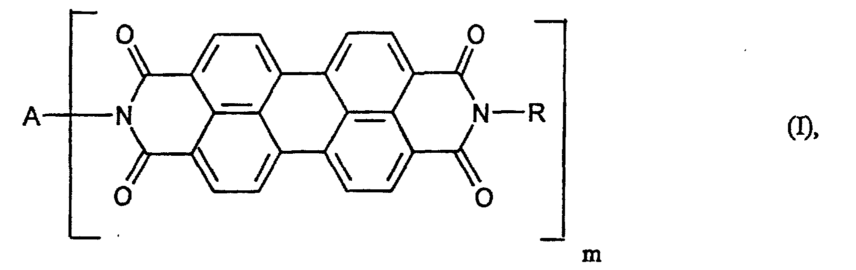 worin A einen zwei-, drei- oder vierwertigen carbocyclischen oder heterocyclischen aromatischen Rest bedeutet, R für H, eine Alkyl-, Aralkyl-, oder Cycloalkylgruppe oder für einen carbocyclischen oder heterocyclischen aromatischen Rest steht und m 2, 3 oder 4 ist, mit der Massgabe, dass wenn m 2 ist und R Phenethyl bedeutet, A nicht 1,4-Phenylen ist.

Die erfindungsgemässen Verbindungen können durch Umsetzung eines Perylen-3,4:9,10-tetracarbonsäure-monoahydrids-monoimids der Formel III 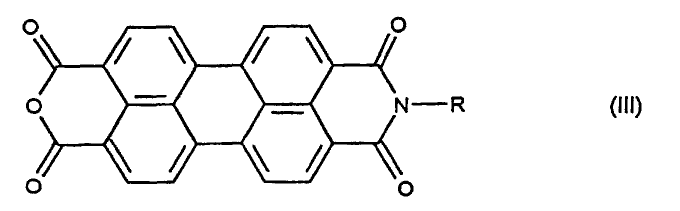 mit einem Formamid der Formel IV

A(NHCHO)ₘ (IV)

hergestellt werden, wobei A, R und m die oben angegebene Bedeutung haben. Dieses Herstellungsverfahren ist ebenfalls Gegenstand vorliegender Erfindung. Vorzugsweise wird die Umsetzung bei einer Temperatur von 150-250°C und in Gegenwart eines stickstoffhaltigen Heterocyclus, einer Carbonsäure oder eines Glykols durchgeführt.

Perylen-3,4:9,10-tetracarbonsäure-monoahydride-monoimide der Formel III sind bekannt oder können nach bekannten Methoden hergestellt werden. Einige dieser Verbindungen sind beispielsweise im oben erwähnten Artikel in Chem. Ber. 124, 529 (1991) beschrieben.

Die zwei-, drei- oder vierwertigen carbocyclischen oder heterocyclischen aromatischen Reste A sind von primären di-, tri- oder tetra-Aminen abgeleitet. Geeignete carbocyclische Amine sind zum Beispiel mono- bis tetracyclische, insbesondere mono- oder bicyclische Amine, vorzugsweise abgeleitet von Benzol, bevorzugt in den Positionen 1,4-, 1,3,5- oder 1,2,4,5-substituiert; von Biphenyl, bevorzugt in den Positionen 4,4'- substituiert oder von Naphthalin, bevorzugt in den Positionen 2,7- substituiert. Geignete heterocyclische aromatische Amine können rein heterocyclisch sein oder einen heterocyclischen Ring und einen oder mehrere ankondensierte Benzolringe enthalten. Beispiele für heterocyclische Ringsysteme sind Pyridin, bevorzugt in den Positionen 2,4- oder 2,4,6-substituiert; Pyrimidin und s-Triazin, jeweils bevorzugt in den Positionen 2,4,6-substituiert.

Bevorzugte Reste A sind von Benzol, Biphenyl, Naphthalin, Anthracen, Phenanthren, Pyridin, Pyridazin, Pyrimidin, Pyrazin oder Triazin abgeleitet, insbesondere Reste der Formel

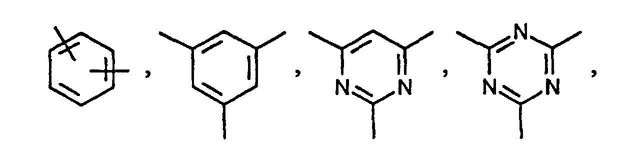

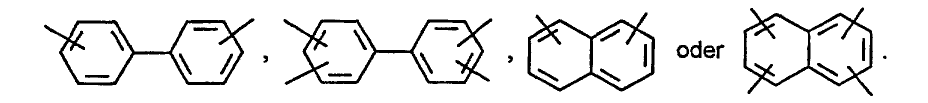

Ferner werden erfindungsgemässe Perylen-3,4:9,10-tetracarbonsäureimide der Formel I bevorzugt, worin R einen sekundären C₇-C₄₁-Alkylrest oder einen Rest der Formel II bedeutet 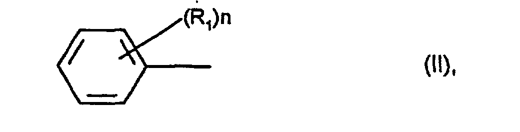 wobei R₁ ein verzweigter C₃-C₈-Alkylrest ist und n für 0, 1, 2 oder 3 steht.

Weitere bevorzugte Verbindungen sind solche, worin R -CH(R₂)₂ bedeutet, und R₂ C₄-C₁₈-Alkyl, vorzugsweise C₆-C₁₀-Alkyl ist, oder worin R einen Rest der oben dargestellten Formel II mit R₁ gleich tert-Butyl bedeutet.

Ganz besonders bevorzugt werden Verbindungen der Formel I, worin R 2,5-Di-tert-butylphenyl oder -CH(R₂)₂ bedeutet und R₂ ein geradkettiger Rest, vorzugsweise n-Hexyl, n-Heptyl, n-Octyl, n-Nonyl oder n-Decyl ist. Ganz besonders bevorzugt sind auch die in den Herstellungsbeispielen beschriebenen Verbindungen.

Ein weiterer Gegenstand der Erfindung ist ein Verfahren zur Herstellung von Perylen-3,4:9,10-tetracarbonsäure-bisimiden der Formel V 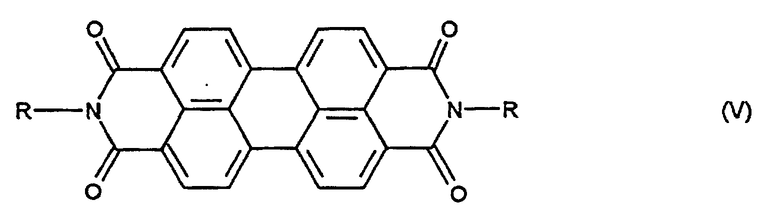 durch Umsetzung des Perylen-3,4:9,10-tetracarbonsäure-bisanhydrids oder von Perylen-3,4:9,10-tetracarbonsäure-monoanhydrid-monoimiden der Formel III 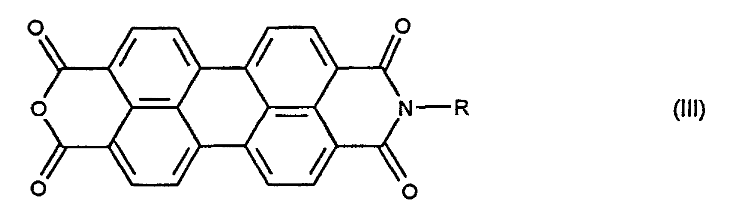 mit einem Formamid der Formel VI, RNHCHO (VI), worin die Reste R unabhängig voneinander H, eine Alkyl-, Aralkyl-, oder Cycloalkylgruppe oder einen carbocyclischen oder heterocyclischen aromatischen Rest bedeuten, dadurch gekennzeichnet, dass die Umsetzung bei einer Temperatur von 150-250°C und in Gegenwart eines stickstoffhaltigen Heterocyclus, einer Carbonsäure oder eines Glykols durchgeführt wird. Bevorzugte Reste R sind die bei den Verbindungen der Formel I oben beschriebenen bevorzugten Reste.

Sowohl das Verfahren zur Herstellung von Verbindungen der Formel I als auch das zuletzt erwähnte Verfahren zur Herstellung von Bisimiden der Formel V werden vorzugsweise in Gegenwart eines stickstoffhaltigen Heterocyclus ausgewählt aus der Gruppe Pyridin, Picolin, Lutidin und insbesondere Imidazol oder Chinolin durchgeführt. Falls die Herstellung in einer Carbonsäure verläuft ist dies vorzugsweise Essigsäure, während das bevorzugte Glykol Ethylenglykol ist.

Die Umsetzung kann ohne weitere Komponenten im Reaktionsgemisch oder geeigneterweise in Gegenwart eines Zink-, Blei-, Calcium- oder Magnesiumsalzes als Katalysator durchgeführt werden. Bevorzugte Katalysatoren sind Bleiacetat, Zinkchlorid oder insbesondere Zinkacetat.

Nicht nur die Reaktionsgeschwindigkeit der Umsetzung, sondern auch die Ausbeuten werden durch Zusatz der oben erwähnten Metallsalze bzw. Katalysatoren erhöht. Optimale Ausbeuten symmetrisch oder unsymmetrisch substituierter Perylenfarbstoffe werden bei Reaktionszeiten von 8-10 h bei einer Temperatur von etwa 180°C unter Zusatz von Zinkacetat erreicht.

Die erfindungsgemässen Verbindungen der Formel I sind vorzugsweise im Perylen-Ringsystem nicht substituiert. Sie können aber auch einen oder mehrere, in der Regel jedoch höchstens sechs Substituenten im Ringsystem aufweisen, wobei die Substituenten unabhängig voneinander Alkyl, Aralkyl, Cycloalkyl, Alkoxy, Aryloxy, Alkylaryl, Alkylmercapto, Arylmercapto, einen carbocyclischen oder heterocyclischen aromatischen Rest oder Chloro, Bromo, Nitro, -SO₃H (sowie deren Metallsalze oder deren Ammoniumsalze) oder -SO₃R (wobei R für Alkyl oder Aryl steht), Amino, Acylaminomethyl, wie z.B. Acetylaminomethyl, Alkylamino, Arylamino, Phthalimidomethyl, Aminomethyl, Dimethylaminomethyl (hergestellt zum Beispiel durch Spaltung des entsprechenden Phthalimido-Derivats), Pyrazolomethyl, sein können.

Die letztgenannten sulfo- bzw. amino-substituierten Perylen-Derivate eignen sich insbesondere als Rheologieverbesserer. Entsprechende Derivate anderer Pigmentsysteme, wie z.B. der Phthalocyaninpigmente oder der Chinacridonpigmente sowie deren Herstellung sind beispielsweise aus US 4,981,888, EP-A 356,390, EP-A 508,704, US5,212,221 oder EP-A 485,337 bekannt. Die vorliegenden substituierten Perylenderivate können auf analoge Weise hergestellt werden.

Vorzugsweise sind der Substituent bzw. die Substituenten in **1**-, bzw. 1,6-, 2,5-, 7,12-, oder 8,11-Stellung. Die substituierten Perylenderivate weisen vorzugsweise einen oder zwei Substituenten im Ringsystem auf, und bei disubstituierten Verbindungen sind die Substituenten vorzugsweise gleich.

Die substituierten Perylenderivate können aus den entsprechenden nicht substituierten Verbindungen nach allgemein bekannten Methoden hergestellt werden.

Die erfindungsgemässen Verbindungen eignen sich anhand ihrer Eigenschaften für eine Vielzahl von Anwendungen.

So können sie beispielsweise als Pigmente für die Massefärbung von Kunststoffen oder Lacken eingesetzt werden. Weitere Gegenstände der Erfindung sind daher in der Masse eingefärbtes hochmolekulares organisches Material enthaltend eine Verbindung der Formel I, sowie ein Verfahren zum Färben von hochmolekularem organischem Material in der Masse unter Verwendung dieser Verbindungen.

Geeignete Kunststoffe sind z.B. Polyolefine, Polyvinylchlorid, Fluorpolymerisate, wie z.B. Polyfluorethylen, Polytrifluorchlorethylen oder Tetrafluorethylen/Hexafluorpropylen-Mischpolymerisat, Silikonharze, insbesondere aber Ingenieurwerkstoffe (Engineering Plastics), wie z.B. Polycarbonate, Polyacrylate, Polymethacrylate, Polystyrol, ABS, Polyester, insbesondere Polyalkylenterephthalate, wie Polybutylenterephthalat (PBT) oder Polyethylenterephthalat (PET), Polyamide, Polyetherketone, Polyurethane, einzeln oder in Mischungen. Zweckmässig werden die erfindungsgemässen Verbindungen in einer Konzentration von 0.01 bis 10, vorzugsweise 0.01-5 Gew.%, bezogen auf das Polymere, eingesetzt.

Als Beispiele für Polyolefine, die mit den erfindungsgemässen Verbindungen gefärbt werden können, seien Polyethylen hoher und niederer Dichte (HD-PE, LD-PE und LLD-PE), Polyisobutylen und insbesondere Polypropylen, sowie Copolymere von Polyolefinen mit z.B. Polyethern, Polyetherketonen oder Polyurethanen, erwähnt. Bevorzugt ist Polypropylen.

Die Färbung erfolgt nach den üblichen Verfahren, beispielsweise durch Mischen einer erfindungsgemässen Verbindung oder eines Gemisches solcher Verbindungen mit dem Kunststoffgranulat oder -pulver, ohne es vorher in ein Präparat einarbeiten zu müssen, und Extrudieren der Mischung zu Fasern, Folien oder Granulaten. Letztere können dann beispielsweise im Spritzgussverfahren zu Gegenständen verformt werden.

Die erhaltenen rot fluoreszierenden Ausfärbungen weisen hohe Reinheit und hohe Sättigung auf und zeichnen sich durch gute Transparenz sowie durch gute Beständigkeit, insbesondere gegen Licht, aus.

Ein weiterer Gegenstand der Erfindung ist die Verwendung der erfindungsgemässen Verbindungen im Sicherheitsdruck, als Fluoreszenzfarbstoffe für maschinenlesbare Markierungen, als Laserfarbstoffe, sowie für die Herstellung von Druck-Tonem ("non-impact printing toners"), Farbfiltern, organischen Photorezeptoren, Elektrolumineszenz- und Photolumineszenzelementen oder Sonnenkollektoren.

Erfindungsgemässe Verbindungen, welche einen oder mehrere Substituenten ausgewählt aus der Gruppe -SO₃H (sowie deren Metallsalze oder deren Ammoniumsalze) oder -SO₃R (wobei R für Alkyl oder Aryl steht), Amino, Acylaminomethyl, wie z.B. Acetylaminomethyl, Alkylamino, Arylamino, Phthalimidomethyl, Aminomethyl, Dimethylaminomethyl (hergestellt zum Beispiel durch Spaltung des entsprechenden Phthalimido-Derivats) oder Pyrazolomethyl aufweisen, können zudem als Rheologieverbesserer eingesetzt werden.

Die nachfolgenden Beispiele erläutern die Erfindung.

### Beispiel 1: Synthese eines bichromophoren Perylenfarbstoffes durch Umsetzung des 2,4,6-Triaminopyrimidins mit N-(1-Hexylheptyl)- perylen-3,4:9,10-tetracarbonsäure-3,4-anhydrid-9,10-carboximid

3.70 g (6.45 mmol) N-(1-Hexylheptyl)-perylen-3,4:9,10- tetracarbonsäure-3,4-anhydrid-9,10-carboximid (hergestellt gemäss H. Kaiser, J. Lindner, H. Langhals, Chem. Ber. 1991, 124, 529) werden in 10 g Imidazol unter Zusatz von 200 mg (0.90 mmol) Zinkacetat und 270 mg (2.16 mmol) 2,4,6,-Triaminopyrimidin 3 h bei 200°C unter Argon- Schutzgas umgesetzt. Das zähflüssige Reaktionsprodukt wird in 500 ml Ethanol aufgenommen, mit 100 ml konz. HCI versetzt und 8 h gerührt. Nach Absaugen über eine D-4 Fritte wird der Farbstoff mit dest. Wasser neutralgewaschen und getrocknet (3.2 g). Er wird über Kieselgel mit CHCl₃/Eisessig 10:1 als Laufmittel chromatographiert, wobei eine geringe Menge eines Vorlaufes verworfen wird. Das rötlich fluoreszierende Produkt wird dabei im ersten Drittel der Säule so fest adsorbiert, dass ein Eluieren auch nach Erhöhung der Eisessig-Konzentration im Lösungsmittel nicht möglich ist. Das intensiv violett gefärbte Kieselgel des ersten Drittels der Säule wird nach Entfernung des Laufmittels und Trocknung der Säulenfüllung mit einem Gemisch von Chloroform und 30 % Eisessig unter Rühren 30 min unter Rückfluss gekocht, wobei der Farbstoff in Lösung geht. Das Gemisch wird nun über eine D-4 Fritte abgesaugt und das verbleibende Kieselgel wird mit Chloroform/Eisessig (10:3) gewaschen. Die intensiv rotviolette Farbstofflösung wird mit dem Rotationsverdampfer eingeengt und der ausfallende Farbstoff nach Zusatz von dest Wasser abgesaugt, neutralgewaschen und 8 h bei 100°C getrocknet. Ausb. 2.3 g (86 %) Rohprodukt. Aus dem erhaltenen Rohprodukt kann durch erneute Chromatographie an Kieselgel mit CHCl₃ unter Zusatz von 10 % Eisessig oder 10 % Ethanol (Säule 80x4 cm) kein elementaranalysenreines Produkt gewonnen werden, auch nicht nach mehrfacher Chromatographie. Ausb. 1.2 g (45%). UV (CHCl₃): λₘₐₓ(∈) = 568 nm (188422), 529 (154867), 492 (90339), 460 (33552). Fluoreszenz (CHCl₃): λₘₐₓ = 532 nm, 575, 620. MS (70eV): m/z(%)= 1234 (0,1), 1052 (0.2), 871 (2), 870 (1), 497 (1), 435 (0.9), 412 (1), 389 (2), 183 (4), 182 (33), 154 (2), 112(7), 111 (16), 98 (15), 97 (39), 85 (9), 84 (31), 83 (59), 82 (13), 70 (65), 69 (100), 55 (89), 43 (40), 28 (9), 27 (13). - MS (FAB/3-NBA/Xe 8KV,10W): m/z= 1254 [M⁺+H₂O], 1237 [M⁺], 681, 596 (100), 520, 499, 414,373, 345, 300, 275, 262, 250, 154, 136, 107. Da das Produkt nicht analysenrein ist, werden 100 mg des Produktgemisches an basischem Al₂O₃ (Aktivität I) mit CHCl₃/n- Butanol 40:1 chromatographiert (Säule 30x1cm). 1.Fraktion: Ausb. 0.64 g (64% bezogen auf eingesetzte Menge). MS (FAB/CHCl₃m-NBA): m/z (%)= 1254 (20), 1237 (100), 1236 (45), 596 (26), 414 (100), 358 (30), 275 (18), 237 (8).

| | | | | |
|---|---|---|---|---|
| C₇₈H₇₃N₇O₈H₂O (1254.4) | Ber. | C 74.76 | H 6.03 | N 7.81 |
| | Gef. | C 74.93 | H 6.28 | N 7.76 |

### Beispiel 2: Synthese eines trichromophoren Perylenfarbstoffes durch Umsetzung des 1,3,5-Triaminobenzol-trisformamid mit N-(1-Hexylheptyl)-3,4:9,10-perylentetracarbonsäure-3,4-anhydrid-9,10-carboximid

(1) 1,3,5-Triaminobenzol wird gemäss Vorschrift in Chem. Abstr. 1954, 49,9036a durch Reduktion von 1,3,5-Trinitrobenzol mit pulverisiertem Eisen in Salzsäure hergestellt. Nach Behandeln mit 2 N HCI erhält man 14.6 g (37%) gelbliche Nadeln - Schmp. 300°C (Hydrochlorid).
(2) 1,3,5-Triaminobenzol-trisformamid
1.0 g (4.3 mmol) 1,3,5-Triaminobenzolhydrochlorid wird in 20 ml konz. Ameisensäure eingetragen und die Lösung wird 30 min zum Sieden erhitzt. Die erkaltete Lösung wird in 250 ml dest. Wasser eingetropft und das ausfallende Produkt wird über eine D-4 Fritte abgesaugt, mit dest. Wasser neutralgewaschen und im Vakuum 2 h bei 50°C getrocknet.
Ausb. 550 mg (62%) graues Pulver- Schmp. 265°C.

| | | | | |
|---|---|---|---|---|
| C₉H₉N₃O₃ (207.2) | Ber. | C 52.17 | H 4.38 | N 20.23 |
| | Gef. | C 51.14 | H 4.43 | N 20.04 |

(3) Synthese des trichromophoren Pervlenfarbstoffes
3.0 g (5.2 mmol) N-(1- Hexylheptyl)-3,4:9,10-perylentetracarbonsäure-3,4-anhydrid-9,10-carboximid (hergestellt gemäss H. Kaiser, J. Lindner, H. Langhals, Chem. Ber. 1991, 124, 529) werden in 12 g Imidazol mit 359 mg (1.73 mmol) 1,3,5- Triaminobenzoltrisformamid unter Argonschutzgas 5 h bei 180°C umgesetzt. Nach dem Erkalten wird die Reaktionsmischung in 1 I Ethanol aufgenommen, mit 100 ml konz. HCI versetzt und 2 h gerührt. Das ausfallende Farbstoffrohprodukt wird durch Absaugen über eine D-4 Fritte abgetrennt, mit dest. Wasser neutralgewaschen und 2 h bei 100°C getrocknet. Ausb. 2.8 g (Rohprodukt). Zur Reinigung wird das Rohprodukt in 30 ml CHCl₃/n-Butanol 10:1 aufgenommen und über Al₂O₃ (Aktivität I) mit CHCl₃/n-Butanol 10:1 chromatographiert. Ein gelbgrün fluoreszierender Vorlauf wird dabei verworfen und die zweite Fraktion, die sich durch eine rote Fluoreszenz auszeichnet, wird abgetrennt. Auf der Säule bleiben eine Reihe von Verunreinigungen im ersten Drittel fest adsorbiert zurück. Die dünnschichtchromatographisch einheitliche Farbstofffraktion wird über eine D-5 Fritte abgesaugt und mit dem Rotationsverdampfer eingedampft. Der ausfallende Farbstoff wird mit dest. Wasser gewaschen und 5h bei 50°C im Vakuum getrocknet. Ausb. 800 mg (26%) - Schmp. 285-300°C (Zersetzung) - R_{f}(Kieselgel/CHCl₃/Eisessig 10:1)= 0.85 - R_{f} (Al₂O₃/CHCl₃/n-BuOH 40:1) 0.52. UV (CHCl₃): λₘₐₓ(∈) = 433 nm (17136), 461 (53243), 492,5 (155141), 530 (298654). Fluoreszenz (CHCl₃):
λₘₐₓ= 538 nm, 578.

| | | | | |
|---|---|---|---|---|
| C₁₁₇H₁₀₈N₆O₁₂H₂O(1808.1) | Ber. | C 77.72 | H 6.13 | N 4.64 |
| | Gef. | C 77.99 | H 6.27 | N 4.84 |

Das verhältnismäßig stabile Farbstoff-Hydrat kann durch 8 h Trocknen bei 100°C im Hochvakuum dehydratisiert werden.

| | | | | |
|---|---|---|---|---|
| C₁₁₇H₁₀₈N₆O₁₂(1790.1) | Ber. | C 78.49 | H 6.08 | N 4.69 |
| | Gef. | C 78.25 | H 6.08 | N 4.84 |

Die Synthese wird mit dem gleichen Ansatz wiederholt, wobei 200 mg Zinkacetat zugesetzt werden. Die Reaktionsdauer wird auf 8 h erhöht und 25 g Imidazol werden als Lösungsmittel verwendet. Es entsteht bei gleicher Aufarbeitung ein Produkt, das identische IR-, ¹H-NMR-, ¹³C-NMR-Spektren aufweist, dessen Elementaranalysenwerte aber stärker abweichen. Die Extinktionskoeffizienten des Produktes sind um 18 % niedriger, was auf eine geringere Reinheit hinweist.

### Beispiel 3: N,N'-(1-Hexylheptyl)-3,4:9,10-perylenbis(dicarboximid)

### (1) N-Hexylheptyl-formamid

20.0 g (100 mmol) 1-Hexylheptylamin werden unter Rückfluss mit 20.0 g (400 mmol) 92 proz. Ameisensäure 1 h erhitzt. Die überschüssige Ameisensäure wird über eine 10 cm Vigreuxkolonne abdestilliert und das verbleibende Reaktionsprodukt wird im Vakuum fraktioniert destilliert. Ausb. 18.1 g (79 %) - Sdp. 165°C(1Torr) - n_{D}20 = 1.4577. Das Reaktionsprodukt enthält einen geringen Anteil an Bisformamid, der destillativ nur schwierig abzutrennen ist, aber bei den weiteren Umsetzungen nicht stört.

| | | | | |
|---|---|---|---|---|
| C₁₄H₂₉NO (227.4) | Ber. | C 73.75 | H 12.86 | N 6.16 |
| | Gef. | C 71.29 | H 12.43 | N 5.94 |

### (2) Synthese des N,N'-(1-Hexylheptyl)-3.4:9,10-perylenbis(dicarboximid) unter Verwendung des Formamids

3.0 g (7.6 mmol) Perylen-3,4:9,10-tetracarbonsäurebisanhydrid werden in 20 g Imidazol mit 200 mg (0.90 mmol) Zinkacetat und 2.60 g (11.4 mmol) Formamid des 1-Hexyl-heptylamins 8 h bei 180°C unter Argonschutzgas gerührt. Ausb. 3.8 g (66%) chromatographiert an Kieselgel mit CHCl₃ (Säule 80x4 cm). Schmp. 157°C - R_{f}(Kieselgel/CHCl₃)= 0.75. UV (CHCl₃): λₘₐₓ(∈) = 460 nm (18239), 492 (52285), 528 (87547). Fluoreszenz (CHCl₃): λₘₐₓ = 538 nm, 573. MS (70eV): m/z (%)= 756 (13), 755 (47), 754 (88) [M⁺], 737 (8), 669 (4), 575 (3), 574 (16), 573 (43), 572 (49), 555 (5),404 (4), 403 (7), 392 (26), 391 (75), 390 (100), 374 (5), 373 (14), 345 (9), 69 (7), 55 (10), 43 (4), 41 (5), 29 (1).

### Beispiel 4: N,N'-Di-phenyl-3,4:9,10-perylenbis(dicarboximid)

2.0 g (5.1 mmol) Perylen-3,4:9,10-tetracarbonsäurebisanhydrid werden in 8 g Imidazol mit 100 mg (0.45 mmol) Zinkacetat und 1.85 g (13.68 mmol) Formanilid unter Argonschutzgas 4 h bei 180°C unter Rühren umgesetzt. Ausb. 2.0 g (72%) extraktiv umkristallisiert aus Methanol.

### Beispiel 5: N-(1-Hexylheptyl)-N'-phenyl)-3,4:9,10-perylen-bis(dicarboximid)

2.00 g (3.48 mmol) N-(1Hexylheptyl)-3,4:9,10-perylentetracarbonsäure-3,4-anhydrid-9,10-carboximid werden mit 847 mg (6.26 mmol) Formanilid und 100 mg (0.45 mmol) Zinkacetat in 10 g Imidazol 10 h bei 180°C unter Argonschutzgas umgesetzt. Das Rohprodukt wird gemäss allgemeiner Vorschrift aufgearbeitet. Ausb. 2.15 g (95%). Zur Reinigung wird der Farbstoff über Kieselgel mit CHCl₃ als Laufmittel chromatographiert (Säule 80x4 cm). Die Farbstofffraktion wird mit dem Rotationsverdampfer eingedampft, der ausfallende Farbstoff wird mit Wasser gewaschen und 4 h bei 50°C im Vakuum getrocknet. Ausb. 1.40 g (62%) - R_{f} (CHCl₃) = 0.53. - UV (CHCl₃): λₘₐₓ(∈) = 492 nm (18750), 492(52272), 528(86727). Fluoreszenz (CHCl₃): λₘₐₓ = 538 nm, 578.

| | | | |
|---|---|---|---|
| C₄₃H₄₀N₂O₄ (648.8) | Ber. C 79.60 | H 6.21 | N 4.31 |
| | Gef. C 79.61 | H 6.28 | N 4.49 |

### Beispiel 6: Synthese eines trichromophoren Perylenfarbstoffes durch Umsetzung des 1,3,5-Triaminobenzol-trisformamid mit N-(1-Heptyloctyl)-3,4:9,10-perylentetracarbonsäure-3.4-anhydrid-9,10-carboximid

1.5 g (2.49 mmol) N-(1- Heptyloctyl)-3,4:9,10-perylentetracarbonsäure-3,4-anhydrid-9,10-carboximid (hergestellt gemäss H. Kaiser, J. Lindner, H. Langhals, Chem. Ber. 1991, 124, 529) werden in 6 g Imidazol mit 172 mg (0.83 mmol) 1,3,5- Triaminobenzoltrisformamid unter Argonschutzgas 5 h bei 185°C umgesetzt Nach dem Erkalten wird die Reaktionsmischung in 0.5 I Ethanol aufgenommen, mit 50 ml konz. HCI versetzt und 2 h gerührt Das ausfallende Farbstoffrohprodukt wird durch Absaugen über eine D-4 Fritte abgetrennt, mit dest. Wasser neutralgewaschen und 2 h bei 120°C getrocknet Ausb. 1.4 g (Rohprodukt). Zur Reinigung wird das Rohprodukt in 15 ml CHCl₃/n-Butanol 10:1 aufgenommen und über Al₂O₃ (Aktivität I) mit CHCl₃/n-Butanol 10:1 chromatographiert. Ein gelbgrün fluoreszierender Vorlauf wird dabei verworfen und die zweite Fraktion, die sich durch eine rote Fluoreszenz auszeichnet, wird abgetrennt. Dieses Produkt wird noch mehrmals chromatographisch gereinigt (CHCl₃/Eisessig 20:1 auf Kieselgel; CHCl₃/Ethanol 10:1 auf Kieselgel; noch einmal CHCl₃/Eisessig 20:1 auf Kieselgel), bis eine dünnschichtchromatographisch einheitliche Farbstofffraktion erhalten wird. Diese wird über eine D-5 Fritte abgesaugt und mit dem Rotationsverdampfer eingedampft. Der ausfallende Farbstoff wird mit dest. Wasser gewaschen und 14h bei 115°C im Vakuum getrocknet. Ausb. 200 mg (13%) - Schmp. 280°C (Zersetzung) - R_{f}(Kieselgel/CHCl₃/Ethanol 10:1)= 0.58. UV (CHCl₃): λₘₐₓ(∈) = 437 nm (13499), 460 (48343), 492 (148632), 530 (294607). Fluoreszenz (CHCl₃): λₘₐₓ= 535 nm, 575.

| | | | | |
|---|---|---|---|---|
| C₁₂₃H₁₂₀N₆O₁₂(1874.3) | Ber. | C 78.82 | H 6.45 | N 4.48 |
| | Gef. | C 79.10 | H 6.74 | N 4.72 |

### Beispiel 7: Synthese eines trichromophoren Perylenfarbstoffes durch Umsetzung des 1,3,5-Triaminobenzol-trisformamid mit N-(1-Nonyldecyl)-3.4:9.10-perylentetracarbonsäure-3,4-anhydrid-9,10-carboximid

Verfährt man analog wie in Beispiel 6, verwendet aber statt N-(1-Heptyloctyl)-3,4:9,10-perylentetracarbonsäure-3,4-anhydrid-9,10-carboximid das entsprechende N-(1-Nonyldecyl)-Derivat, erhält man mit 1,3,5-Triaminobenzol-trisformamid N-(1-Nonyldecyl)-3,4:9,10-perylentetracarbonsäure-3,4-anhydrid-9,10-carboximid.

## Patentansprüche

1. Perylen-3,4:9,10-tetracarbonsäureimid der Formel I 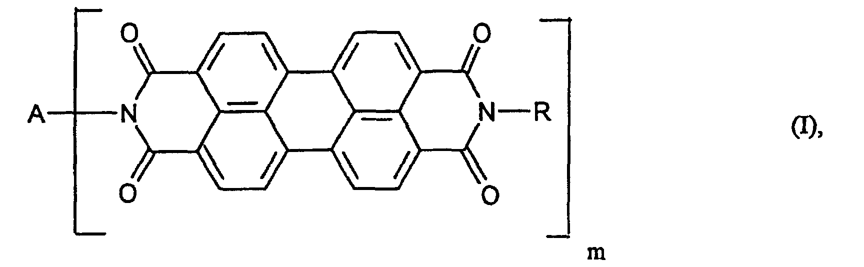 worin A einen zwei-, drei- oder vierwertigen carbocyclischen oder heterocyclischen aromatischen Rest bedeutet, R für H, eine Alkyl-, Aralkyl-, oder Cycloalkylgruppe oder für einen carbocyclischen oder heterocyclischen aromatischen Rest steht und m 2, 3 oder 4 ist, mit der Massgabe, dass wenn m 2 ist, und R Phenylethyl bedeutet, A nicht 1,4-Phenylen ist.

2. Perylen-3,4:9,10-tetracarbonsäureimid nach Anspruch 1, worin R einen sekundären C₇-C₄₁-Alkylrest oder einen Rest der Formel II bedeutet 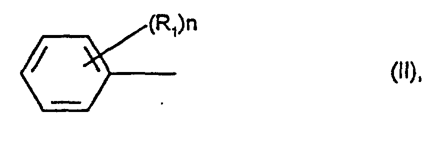 worin R₁ ein verzweigter C₃-C₈-Alkylrest ist und n 0, 1, 2 oder 3 bedeutet.

3. Perylen-3,4:9,10-tetracarbonsäureimid nach Anspruch 1 oder 2, worin R -CH(R₂)₂ bedeutet, und R₂ C₄-C₁₈-Alkyl, vorzugsweise C₆-C₁₀-Alkyl ist, oder worin R einen Rest der Formel II mit R₁ gleich tert-Butyl bedeutet.

4. Perylen-3,4:9,10-tetracarbonsäureimid nach einem der Ansprüche 1 bis 3, worin R 2,5-Di-tert-butylphenyl oder -CH(R₂)₂ bedeutet und R₂ ein geradkettiger Rest, vorzugsweise n-Hexyl, n-Heptyl, n-Octyl, n-Nonyl oder n-Decyl, ist.

5. Perylen-3,4:9,10-tetracarbonsäureimid nach einem der Ansprüche 1 bis 4, worin A ein von Benzol, Biphenyl, Naphthalin, Anthracen, Phenanthren, Pyridin, Pyridazin, Pyrimidin, Pyrazin oder Triazin abgeleiteter Rest ist.

6. Perylen-3,4:9,10-tetracarbonsäureimid nach einem der Ansprüche 1 bis 5, worin A einen Rest der Formel 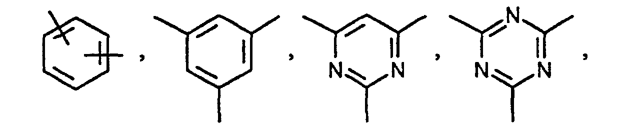 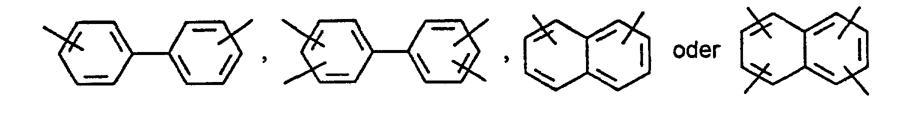 darstellt.

7. Verfahren zur Herstellung eines Perylen-3,4:9,10-tetracarbonsäureimids der Formel I nach Anspruch 1 durch Umsetzung eines Perylen-3,4:9,10-tetracarbonsäure-monoahydridsmonoimids der Formel III 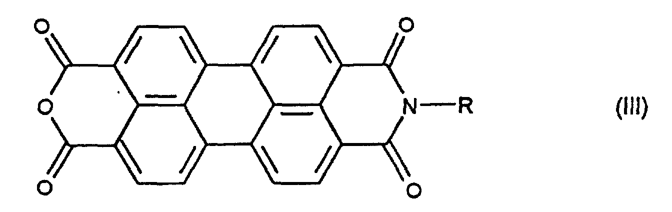 mit einem Formamid der Formel IV
A(NHCHO)ₘ (IV)
wobei A, R und m die im Anspruch 1 angegebene Bedeutung haben.

8. Verfahren nach Anspruch 7, worin die Umsetzung bei einer Temperatur von 150-250°C und in Gegenwart eines stickstoffhaltigen Heterocyclus, einer Carbonsäure oder eines Glykols durchgeführt wird.

9. Verfahren zur Herstellung eines Perylen-3,4:9,10-tetracarbonsäure-bisimids der Formel V 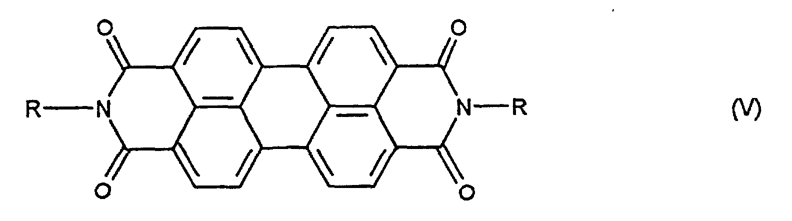 durch Umsetzung des Perylen-3,4:9,10-tetracarbonsäure-bisanhydrids oder von Perylen-3,4:9,10-tetracarbonsäure-monoanhydrid-monoimiden der Formel III 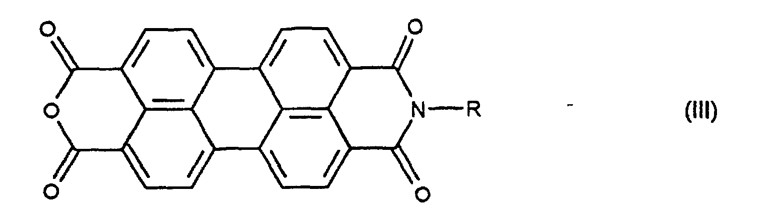 mit einem Formamid der Formel Vl, RNHCHO (Vl), worin die Reste R unabhängig voneinander H, eine Alkyl-, Aralkyl-, oder Cycloalkylgruppe oder einen carbocyclischen oder heterocyclischen aromatischen Rest bedeuten, **dadurch gekennzeichnet**, dass die Umsetzung bei einer Temperatur von 150-250°C und in Gegenwart eines stickstoffhaltigen Heterocyclus, einer Carbonsäure oder eines Glykols durchgeführt wird.

10. Verfahren nach einem der Ansprüche 7 bis 9, worin der stickstoffhaltige Heterocyclus Pyridin, Picolin, Lutidin und insbesondere Imidazol oder Chinolin ist, die Carbonsäure Essigsäure und das Glykol Ethylenglykol ist.

11. Verfahren nach einem der Ansprüche 7 bis 10, worin die Umsetzung in Gegenwart eines Zink-, Blei-, Calcium- oder Magnesiumsalzes, vorzugsweise Bleiacetat, Zinkchlorid oder insbesondere Zinkacetat, durchgeführt wird.

12. In der Masse eingefärbtes hochmolekulares organisches Material enthaltend ein Perylen-3,4:9,10-tetracarbonsäureimid nach einem der Ansprüche 1 bis 6.

13. Verfahren zum Färben von hochmolekularem organischem Material in der Masse, gekennzeichnet durch Verwendung einer Verbindung nach einem der Ansprüche 1 bis 6.

14. Verwendung einer Verbindung nach einem der Ansprüche 1 bis 6 im Sicherheitsdruck, als Fluoreszenzfarbstoffe für maschinenlesbare Markierungen, als Laserfarbstoffe, sowie für die Herstellung von Druck-Tonem ("non-impact printing toners"), Farbfiltern, organischen Photorezeptoren, Elektrolumineszenz- und Photolumineszenzelementen oder Sonnenkollektoren.

## Claims

1. A perylene-3,4:9,10-tetracarboxylic acid imide of the formula I 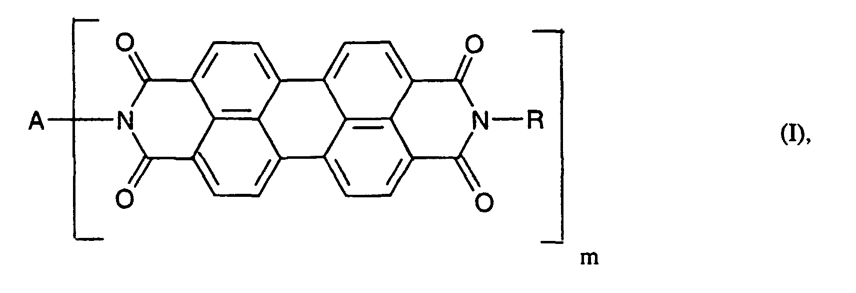 in which
A is a di-, tri- or tetravalent carbocyclic or heterocyclic aromatic radical,
R is H, an alkyl, aralkyl or cycloalkyl group or a carbocyclic or heterocyclic aromatic radical and
m is 2, 3 or 4,
with the proviso that if m is 2 and R is phenethyl, A is not 1,4-phenylene.

2. A perylene-3,4:9,10-tetracarboxylic acid imide according to claim 1, in which
R is a secondary C₇-C₄₁ alkyl radical or a radical of the formula II 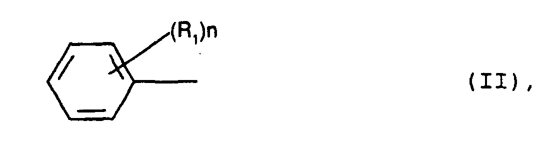 in which
R₁ is a branched C₃-C₈-alkyl radical and
n is 0, 1, 2 or 3.

3. A perylene-3,4:9,10-tetracarboxylic acid imide according to claim 1 or 2,
in which
R is -CH(R₂)₂ and
R₂ is C₄-C₁₈- alkyl, preferably C₆-C₁₀-alkyl,
or in which
R is a radical of the formula II
in which
R₁ is tert-butyl

4. A perylene-3,4:9,10-tetracarboxylic acid imide according to any one of claims 1 to 3,
in which
R is 2,5-di-tert-butylphenyl or -CH(R₂)₂ and
R₂ is a straight-chain radical, preferably n-hexyl, n-heptyl, n-octyl, n-nonyl or n-decyl.

5. A perylene-3,4:9,10-tetracarboxylic acid imide according to any one of claims 1 to 4,
in which
A is a radical derived from benzene, biphenyl, naphthalene, anthracene, phenanthrene, pyridine, pyridazine, pyrimidine, pyrazine or triazine.

6. A perylene-3,4:9,10-tetracarboxylic imide according to any one of claims 1 to 5,
in which
A is a radical of the formula

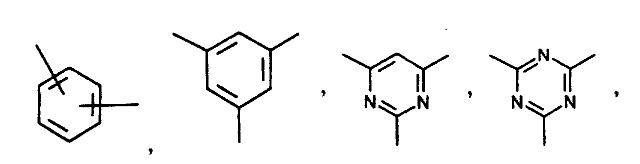

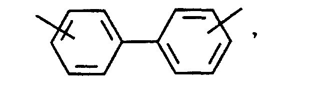

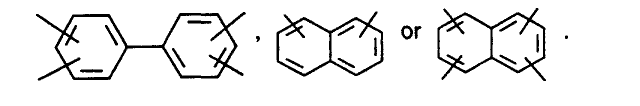

7. A process for the preparation of a perylene-3,4:9,10-tetracarboxylic acid imide of the formula I according to claim 1 by reaction of a perylene-3,4:9,10-tetracarboxylic acid monoanhydride monoimide of the formula III 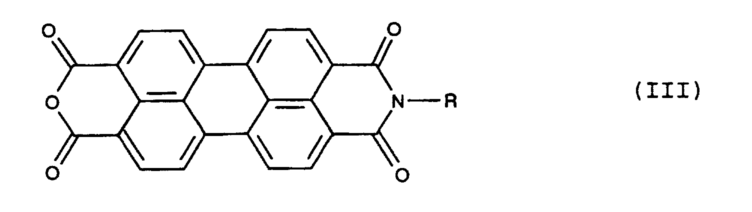 with a formamide of the formula IV
A(NHCHO)ₘ (IV)
in which
A, R and m are as defined in claim 1.

8. A process according to claim 7, wherein the reaction is carried out at a temperature of 150-250°C and in the presence of a nitrogen-containing heterocyclic compound, a carboxylic acid or a glycol.

9. A process for the preparation of a perylene-3,4:9,10-tetracarboxylic acid bisimide of the formula V 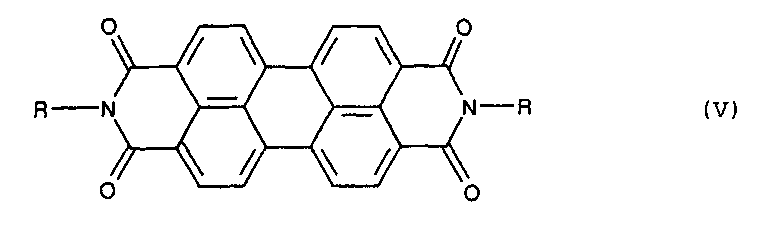 by reaction of perylene-3,4:9,10-tetracarboxylic acid bisanhydride or a perylene-3,4:9,10-tetracarboxylic acid monoanhydride imide of the formula III 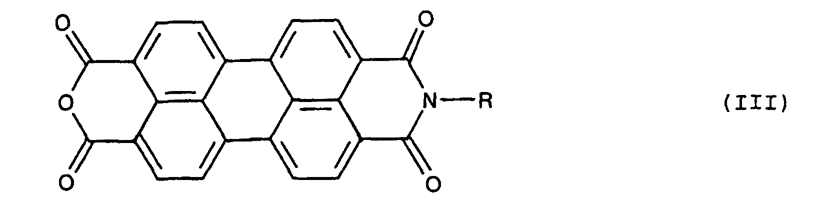 with a formamide of the formula VI, RNHCHO (VI),
in which
the radicals R independently of one another are H, an alkyl, aralkyl or cycloalkyl group or a carbocyclic or heterocyclic aromatic radical,
which comprises carrying out the reaction at a temperature of 150-250°C and in the presence of a nitrogen-containing heterocyclic compound, a carboxylic acid or a glycol.

10. A process according to any one of claims 7 to 9, wherein the nitrogen-containing heterocyclic compound is pyridine, picoline, lutidine or, in particular, imidazole or quinoline, the carboxylic acid is acetic acid and the glycol is ethyleneglycol.

11. A process according to any one of claims 7 to 10, wherein the reaction is carried out in the presence of a zinc, lead, calcium or magnesium salt, preferably lead acetate, zinc chloride or, in particular, zinc acetate.

12. Melt-coloured high molecular weight organic material comprising a perylene-3,4:9,10-tetracarboxylic acid imide according to any one of claims 1 to 6.

13. A process for melt coloration of high molecular weight organic material, which comprises using a compound according to any one of claims 1 to 6.

14. The use of a compound according to any one of claims 1 to 6 in security printing, as a fluorescence dye for machine-readable markings, as a laser dye and for the production of printing toners ("non-impact printing toners"), colour filters, organic photoreceptors, electroluminescence and photoluminescence elements or solar collectors.

## Revendications

1. Pérylène-3,4:9,10-tétracarboximide de formule I 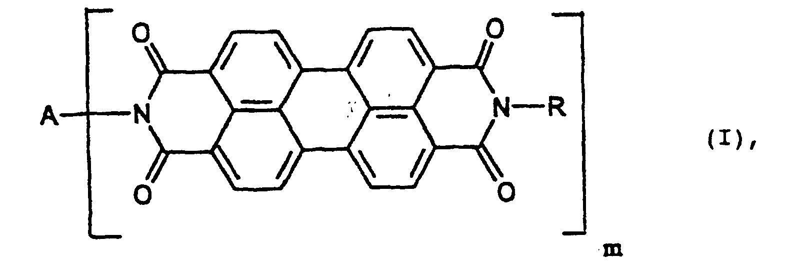 où A représente un reste aromatique carbocyclique ou hétérocyclique bi-, tri- ou tétravalent, R représente un atome d'hydrogène, un groupe alkyle, aralkyle ou cycloalkyle ou un reste aromatique carbocyclique ou hétérocyclique et m vaut 2, 3 ou 4, à condition que lorsque m vaut 2 et R représente un groupe phénéthyle, A ne représente pas 1,4-phénylène.

2. Pérylène-3,4:9,10-tétracarboximide selon la revendication 1, où R représente un reste alkyle en C₇-C₄₁ secondaire ou un reste de formule II 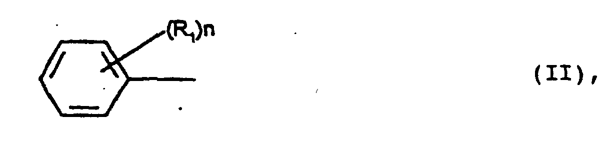 où R₁ représente un reste alkyle en C₃-C₈ et n vaut 0, 1, 2 ou 3.

3. Pérylène-3,4:9,10-tétracarboximide selon la revendication 1 ou 2, où R représente un groupe -CH(R₂)₂, et R₂ représente un groupe alkyle en C₄-C₁₈, de préférence alkyle en C₆-C₁₀, ou dans lequel R représente un reste de formule II avec R₁ égal à tert-butyle.

4. Pérylène-3,4:9,10-tétracarboximide selon l'une des revendications 1 à 3, où R représente un groupe 2,5-di-tert-butylphényle ou -CH(R₂)₂, et R₂ représente un reste à chaîne droite, de préférence n-hexyle, n-heptyle, n-octyle ou n-décyle.

5. Pérylène-3,4:9,10-tétracarboximide selon l'une des revendications 1 à 4, où A représente un reste dérivant des benzène, biphényle, naphtalène, anthracène, phénanthrène, pyridine, pyridazine, pyrimidine, pyrazine ou triazine.

6. Pérylène-3,4:9,10-tétracarboximide selon l'une des revendications 1 à 5, où A représente un reste de formule

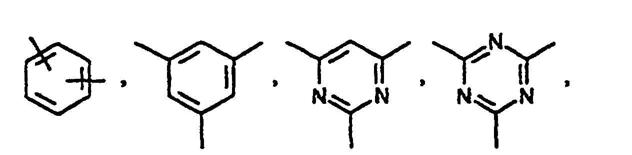

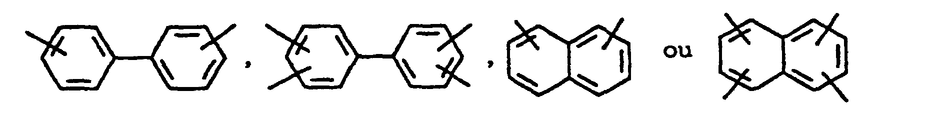

7. Procédé pour la préparation d'un pérylène-3,4:9,10-tétracarboximide de formule I selon la revendication 1 par réaction d'un pérylène-3,4:9,10-tétracarboxy-monoanhydride-monoimide de formule III 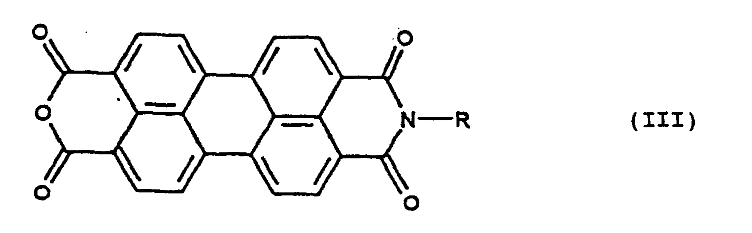 avec un formamide de formule IV
A(NHCHO)ₘ (IV)
où A, R et m possèdent la signification donnée à la revendication 1.

8. Procédé selon la revendication 7, où on met en oeuvre la réaction à une température de 150 à 250°C et en présence d'un composé hétérocyclique azoté, d'un acide carboxylique ou d'un glycol.

9. Procédé pour la préparation d'un pérylène-3,4:9,10-tétracarboxy-bisimide de formule V 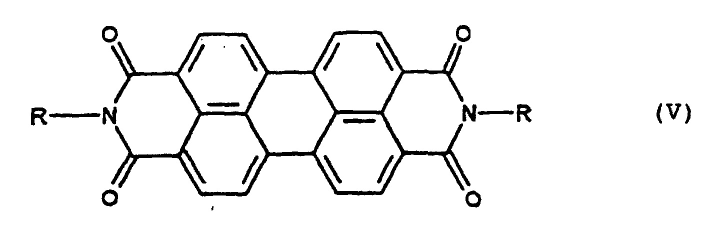 par réaction du bisanhydride pérylène-3,4:9,10-tétracarboxylique ou de pérylène-3,4:9,10-tétracarboxymonoanhydride-monoimides de formule III 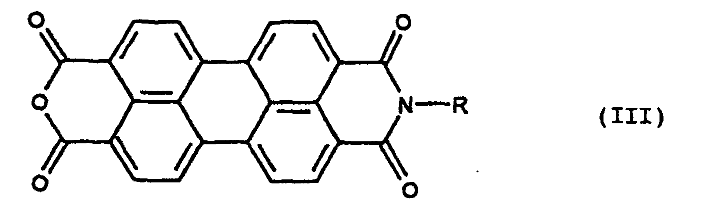 avec un formamide de formule VI, RNHCHO (VI), où les restes R représentent, indépendamment l'un de l'autre, un atome d'hydrogène, un groupe alkyle, aralkyle ou cycloalkyle ou un reste aromatique carbocyclique ou hétérocyclique, **caractérisé en ce qu**'on met en oeuvre la réaction à une température de 150 à 250°C et en présence d'un composé hétérocyclique azoté, d'un acide carboxylique ou d'un glycol.

10. Procédé selon l'une des revendications 7 à 9, où le composé hétérocyclique azoté est la pyridine, la picoline, la lutidine et en particulier l'imidazole ou la quinoléine, l'acide carboxylique est l'acide acétique ou le glycol est l'éthylèneglycol.

11. Procédé selon l'une des revendications 7 à 10, où on met en oeuvre la réaction en présence d'un sel de zinc, de plomb, de calcium ou de magnésium, de préférence de l'acétate de plomb, du chlorure de zinc ou en particulier de l'acétate de zinc.

12. Matière organique de haut poids moléculaire colorée dans la masse contenant un pérylène-3,4:9,10-tétracarboximide selon l'une des revendications 1 à 6.

13. Procédé pour la coloration dans la masse de matière organique de haut poids moléculaire, caractérisé par l'utilisation d'un composé selon l'une des revendications 1 à 6.

14. Utilisation d'un composé selon l'une des revendications 1 à 6 dans l'impression de sécurité en tant que colorants fluorescents pour des marquages lus par machine, en tant que colorants laser ainsi que pour la préparation de "non-impact printing toners", de filtres colorés, de photo-récepteurs organiques, d'éléments d'affichage électro-luminescents et photoluminescents ou de collecteurs d'énergie solaire.
